# EUROPEAN PATENT SPECIFICATION

(11) **EP 1 807 178 B1**
(45) Date of publication and mention of the grant of the patent: **05.12.2012**
(21) Application number: 05804193.0
(22) Date of filing: 04.10.2005
(51) Int. Cl.: B01D 63/08, B01D 19/00, B41J 2/175, B41J 2/19, A61M 1/16, B01D 69/06

(54) **LIQUID-GAS SEPARATOR**
FLÜSSIGKEITS-/GASABSCHEIDER
SEPARATEUR LIQUIDE-GAZ

(30) Priority: 22.10.2004 US 621153 P; 29.10.2004 US 977277
(43) Date of publication of application: 18.07.2007
(73) Proprietor: HEWLETT-PACKARD DEVELOPMENT COMPANY, L.P., Houston, TX 77070 (US)
(72) Inventor: KENT, Blair, M., Vancouver, Washington 98683 (US)
(74) Representative: Stöckeler, Ferdinand
(86) International application number: PCT/US2005/035779
(87) International publication number: WO 2006/047053

(56) References cited:
- EP-A- 0 973 031
- EP-A- 1 398 299
- WO-A-2004/004807
- US-A- 3 523 408
- US-A- 4 642 098
- US-A- 5 439 587
- US-A- 5 659 346

## Description

### BACKGROUND

Certain fluid delivery devices need to remove air or other gas from the liquid before it is delivered to a destination. By way of example, in printing devices, such as, inkjet printers, it is desirable to remove air and/or other gases from ink that is being supplied to a printhead because the printhead may malfunction when air or other gases interfere with its operation. Another exemplary liquid delivery device is an intravenous drug/liquid delivery device, wherein it is desirable to remove air or other gases prior to delivering the drug/liquid to a patient.

To remove air or other gas from a liquid, these and other like liquid delivery devices typically use a purging mechanism that separates the air/gas from the liquid. Such purging mechanisms are typically designed to operate in a particular orientation and as such may fail to operate correctly if their orientation changes. It would be desirable to have a liquid gas separator that can operate in a variety of different orientations without failing.
US 5439587 A relates to a self-priming filter holder for filtration of intravenous liquids, which is formed of one or two housing portions sealed to a core portion. The core portion has one or two ribbed surfaces forming a plurality of flow paths sealed at one end and open at a second end and at least one channel preferably larger than the flow paths. The channel(s) is in fluid communication with the flow paths and an outlet. An inlet is provided to the interior of the housing portion(s) which are sealed from the ribbed surfaces by hydrophilic membranes. The housing portion(s) can be provided with gas vents sealed with hydrophobic membranes. Fluids pass through the flow paths in one direction and through the channel(s) in an opposite direction to remove gas from the flow paths and channels prior to use.
WO 2004/004807 A discloses an infusion device for medical use, comprising a container designed to hold a specified quantity of a liquid to be infused into a patient, a weighing device associated for operation with the container to measure the weight of the container and emit a corresponding control signal, a transport line connected to the container, a pump and a control unit associated with the weighing device and with the pump. The control unit is capable of detecting the end of infusion condition and of commanding the pump to stop the transport of fluid. A separator for continuously separating fluid into a gaseous portion and a liquid portion operates in the infusion line downstream of the pump and prevents the infusion of gas during the stopping transient of the pump at the end of infusion.
US 4642098 A discloses an IV filter and pump assembly having first and second bodies secured into a unitary assembly. The first and second bodies provide a chamber. One of the bodies has an opening therein in communication with said chamber and an inlet port therein. The other of the bodies has an outport therein. The inlet and outlet ports are in communication with the chamber. An inlet valve controls the flow of liquid through the inlet port and an outlet valve controls the flow of liquid through the outlet port. A hydrophobic filter is carried by the unitary assembly and is in communication with the upper portion of said chamber for venting to ambient small quantities of air in said chamber. An actuator supplies a pumping action to the liquid in the chamber so that liquid is caused to be moved out of the chamber through the outlet valve and into the chamber through the inlet valve.

### BRIEF DESCRIPTION OF THE DRAWINGS

The following detailed description refers to the accompanying figures. In the figures, the left-most digit(s) of a reference number identifies the figure (Fig.) in which the reference number first appears. Moreover, the same reference numbers are used throughout the drawings to reference like features and components.

Fig. 1 is a block diagram depicting an exemplary liquid delivery device having a liquid-gas separator, in accordance with certain embodiments of the present invention.

Figs. 2A and 2B are illustrative diagrams depicting a cross-sectional view of an exemplary liquid-gas separator, in accordance with comparative examples useful in understanding the invention.

Fig. 3 is an illustrative diagram depicting a cross-sectional view of an exemplary liquid-gas separator, in accordance with an embodiment of the present invention.

Figs. 4A-E are illustrative diagrams depicting exemplary shapes for a gas-permeable membrane and a liquid-permeable membrane for use in a liquid-gas separator, in accordance with certain different embodiments of the present invention.

Fig. 5 is a block diagram depicting an exemplary printing device having a liquid delivery device that includes a liquid-gas separator, in accordance with certain embodiments of the present invention.

Figs. 6A-B are illustrative diagrams depicting cross-sectional views of two exemplary liquid-gas separators in accordance with a comparative example (Fig. 6A) and in accordance with in other embodiment of the present invention (Fig. 6B).

Fig. 7 is an illustrative diagram depicting a test drop height measurement technique from a side view and a top view.

Fig. 8 is an illustrative diagram depicting a cross-sectional view of a gas-permeable membrane having a plurality of layers, in accordance with certain further embodiments of the present invention.

### DETAILED DESCRIPTION

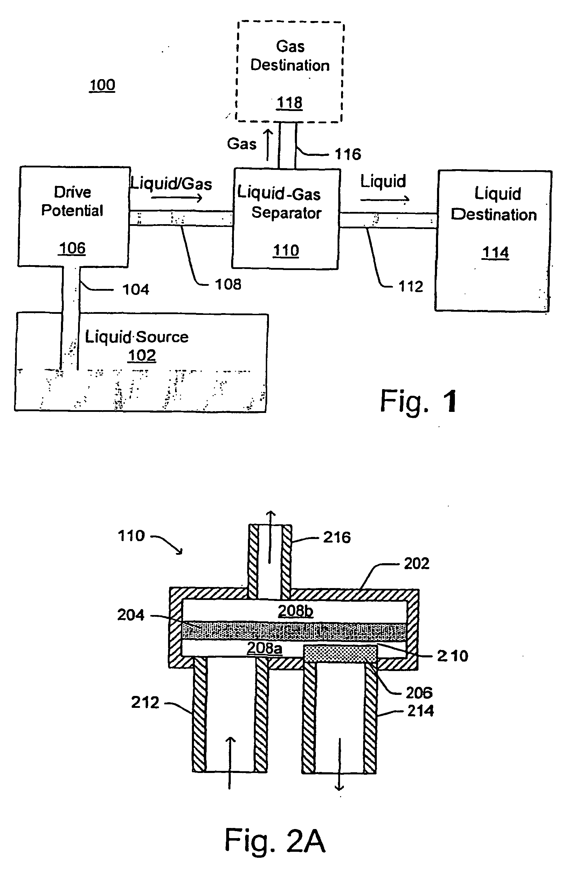

Fig. 1 is a block diagram depicting an exemplary liquid delivery device 100, in accordance with certain embodiments of the present invention.

Liquid delivery device 100 includes a liquid source 102 that is configured to hold at least one liquid. Liquid source 102 is coupled a drive potential 106 through a conduit 104 in a manner that allows the liquid held in liquid source 102 to be withdrawn via conduit 104. Drive potential 106 is representative of a variety of mechanisms that urge the withdrawal of liquid from liquid source 102 through conduit 104 and then into conduit 108. By way of example, drive potential 106 may include a pump or the like. In certain implementations, drive potential 106 may include an arrangement that employs gravity to urge the movement of the liquid.

Conduit 108 is further coupled to an inlet of a liquid-gas separator 110. Liquid-gas separator 110 is configured to at least substantially separate gas that may be present in the urged flowing liquid. The separated gas exit liquid-gas separator 110 through a gas outlet. In this example, a conduit 116 directs the gas or gasses to an optional gas destination 118 that collects or otherwise processes the gas in some manner. In certain implementations, gas destination 118 may be configured to return the gas to liquid source 102 or into another component of device 100. In other examples, conduit 116 and/or the gas outlet may be configured to simply release the gas into the atmosphere.

Liquid-gas separator 110 also includes a liquid outlet that is coupled to conduit 112. The liquid having been separated from the gas continues to be urged by drive potential 106 through conduit 112 to at least one liquid destination 114.

Conduits 104, 108, 112, and 116 are representative of one or more structures or other arrangements that allow the urging by drive potential 106 of the liquid or liquid-gas mixture to occur. By way of example, in certain implementations such conduits may include tubes, pipes, channels, guides, filters, connectors, valves, gauges, sensors, heaters, etc.

Fig. 1 has been illustrated, through the use of gray shading, to better show the flow of liquid (shaded) and gas (non-shaded) within device 100. As shown by the breaks in the shading within conduits 104 and 108, gas may become mixed with the liquid. Fluid-gas separator 110 separates the gas from the liquid as illustrated by the continuous shading within conduit 112.

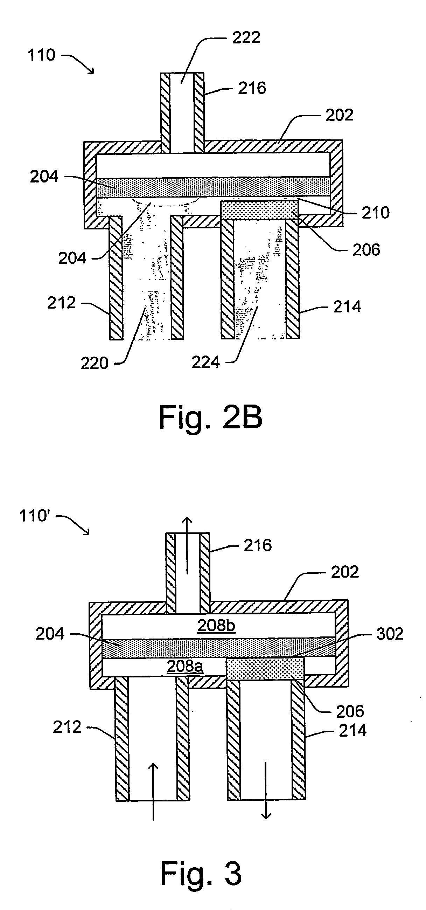

Figs. 2A and 2B are illustrative diagrams depicting a cross-sectional view of an exemplary liquid-gas separator 110 useful in understanding the present invention.

As shown in Fig. 2A, liquid-gas separator 110 includes a body or housing 202 having an inlet 212 through which a liquid and gas mixture can flow into a chamber 208a within housing 202. Chamber 20 8a is separated by a chamber 208b by a gas-permeable membrane 204. Gas-permeable membrane 204 is configured to allow gas within chamber 208a to pa ss through membrane 204 and enter chamber 208b. Gas-permeable membrane 204 is configured to not allow liquid within chamber 208a to enter chamber 208b. Gas that passes through gas-permeable membrane 204 and into chamber 208b may then exit separator 110 via gas outlet 216

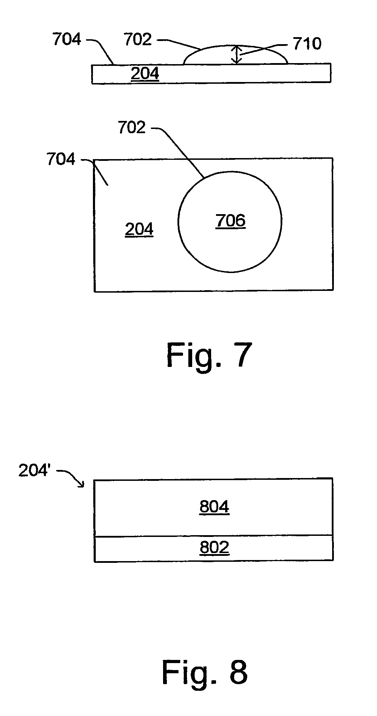

Gas-permeable membrane materials are well known. Gas-permeable membrane 204 may include, for example, a hydrophobic material, an oleophobic material, or the like. As depicted in Fig. 8, gas-permeable membrane 204' may also include two or more layers of materials, such as, an interface layer 802 and a backing layer 804. Such layers may be bonded or otherwise held together. Here, interface layer 802 is configured to allow the gas to pass through it but not the liquid as described above, and backing layer 804 is configured to provide structural support to interface layer 802 while also allowing the gas to pass therethrough. Note that Fig, 8 is illustrative only and hence the layers are not necessarily drawn to scale.

Gas-permeable membrane 204/204' may include, for example, a "breathable" or microporous material such as a fabric, membrane, laminate, etc, made from polytetrafluoroethylene (PTFE), expanded PTFE, porous PTFE, or other like materials. One example, of such materials includes GORE-TEX ™ ePTFE based membrane material, currently sold for packaging vents in a laminate form by W. L. Gore and Associates, Inc. of Newark, Delaware. This is just one example; those skilled in the art will recognize that other types of gas-permeable materials may also be used.

Fluid within chamber 208a is urged through a liquid-permeable membrane 206. liquid-permeable membrane 206 is configured to allow liquid to pass through it from chamber 208a into a liquid outlet 214. Once properly wetted, liquid-permeable membrane 206 is configured to not allow gas to pass through it from chamber 208a into a liquid outlet 214. instead, the gas within chamber 208a will pass through gas-permeable membrane 204 into chamber 208b as described above.

Liquid-permeable membrane 206 may include any material that exhibits appropriate liquid-permeability and gas-impermeability properties when wetted. Liquid-permeable membrane 206 may, for example, include hydrophilic, oleophilic, or other like materials. Liquid-permeable membrane 206 may include one or more materials in one or more layers. By way of example, liquid-permeable membrane 206 may include fabric, a screen, a mesh, or the like with openings sized to allow liquid to pass therethrough but not gaseous bubbles once wetted.

In accordance with certain aspects of the present invention, once gas-liquid separator 110 is properly primed with the liquid/gas mixture, the amount of pressure (e.g., bubble pressure) needed to force the gas through gas-permeable membrane 204 is less than the amount of pressure needed to force the gas through wetted fluid-permeable membrane 206. Conversely, while gas-liquid separator 110 is primed and operating, the amount of pressure needed to force the liquid through wetted liquid-permeable membrane 206 is less than the amount of pressure needed to force the liquid through non-wetted gas-permeable membrane 204.

In this example, a portion of membranes 204 and 206 are positioned adjacent one another within chamber 208a with a small gap 210 separating them. Gap 210 is small enough to prevent the gas within chamber 208a from forming one or more bubbles or a layer that significantly or completely covers liquid-permeable membrane 206. If such were to occur, then it is possible that the urged liquid may force some of the gas through liquid-permeable membrane 206. Gap 210 may be sized, therefore, based on any number of factors including, for example, the type of liquid(s), the type of gas(s), membrane characteristics, liquid pressures, etc.

The size of gap 210 may be determined, for example, by testing gas-permeable membrane 204 using the liquid as illustrated In Fig. 7. The upper drawing shows a side view and the lower drawing shows a top view. Here, a test drop 702 of the liquid is placed onto a non-wetted surface 704 of gas-permeable membrane 204. Test drop 702, in this example, covers an area 706 that is about the same size as a corresponding area of liquid-permeable membrane 206. A test drop height 710 of test drop 702 is then measured. Test drop height 710 may then be considered to represent a maximum size (distance) for gap 210, for example, should separator 110 be intended for operations in different orientations. In certain implementations, gap 210 may therefore be sized to be less than test drop height 710.

Fig. 3 is an illustrative diagram depicting a cross-sectional view of an exemplary liquid-gas separator 110', in accordance with an embodiment of the present invention. Liquid-gas separator 110' is similar to liquid-gas separator 110 of Figs. 2A-B, with the exception that gap 210 between gas-permeable membrane 204 and liquid-permeable membrane 206 no longer exists. Instead, gas-permeable membrane 204 and liquid-permeable memb rane 206 are actually in physical contact with one another, forming contact interface 302. In certain embodiments, gas-permeable membrane 204 may be configured to flex or otherwise move in response to liquid pressure within chamber 208a thereby opening contact interface 302 in such a manner to allow fluid to flow from chamber 208a through liquid-permeable membrane 206.

In accordance with certain aspects of the present invention, liquid-gas separators 110 and 110' can be configured to operate in multiple, it not all, orientations by providing a contact interface 302. In such a configuration gas bubbles should come into co ntact with gas-permeable membrane 204 before or at about the same time that they would contact liquid-permeable membrane 206. As a result, the gas will flow through gas-permeable membrane, which is configured to provide a lower resistance for gas flow than liquid-permeable membrane 206. Thus, as pressure builds or is applied by the urging of drive potential 106 within chamber 208a the gas will be forced out of the mixture through gas-permeable membrane 204.

The exemplary separators of Figs. 2A-B and Fig. 3 illustrate membranes 204 and 206 has having a substantially planer shape. It should be understood, however, that one or both of these membranes may have a non-planer shape. Furthermore, the size and/or surface area of one or more of these membranes may vary depending upon the application. Thus, in certain implementations, membrane 206 may be larger than membrane 204. Also, in certain implementations there may be more than one gas-permeable membrane, and/or more than one liquid-permeable membrane.

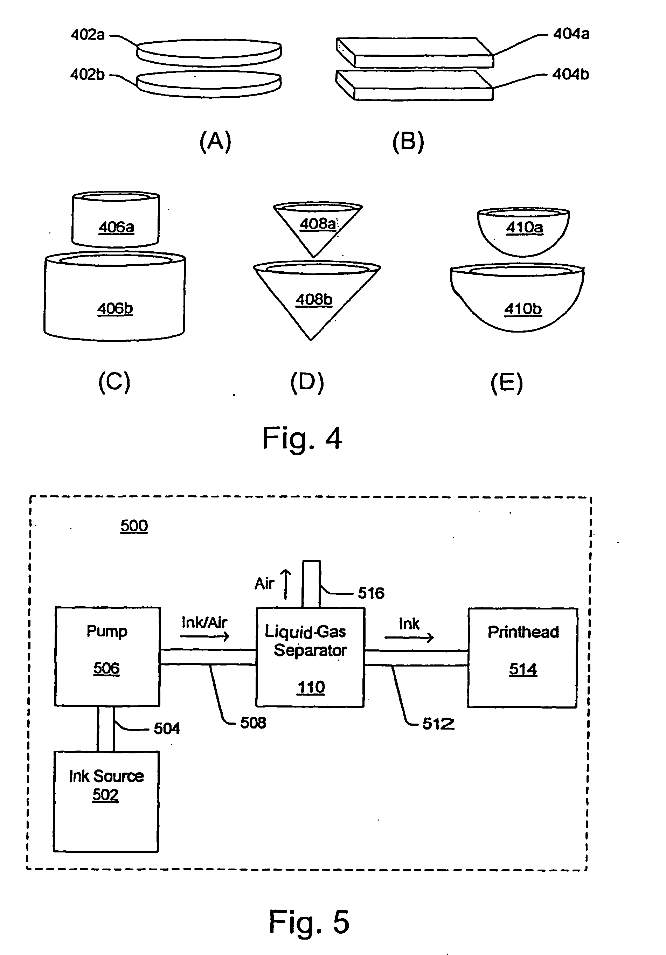

Some exemplary shapes for membranes 204 and/or 206 are illustrated in Figs. 4A-E, in accordance with certain different embodiments of the present invention. Fig. 4A depicts a substantially planer disk shaped gas-permeable membrane 402a and a substantially planer disk shaped liquid-permeable membrane 402b. Fig. 4B depicts a substantially planer rectangular shaped gas-permeable membrane 404a and a substantially planer rectangular shaped liquid-permeable membrane 404b. Fig. 4C depicts a cylindrically shaped gas-permeable membrane 406a and a cylindrically shaped liquid-permeable membrane 406b. Fig. 4D depicts a conically shaped gas-permeable membrane 408a and a conically shaped liquid-permeable membrane 408b. Fig. 4E depicts a spherically shaped gas-permeable membrane 410a and a spherically shaped liquid-permeable membrane 410b.

Fig. 5 is a block diagram depicting an exemplary printing device 500 having a liquid-gas separator 110 (or 110'), in accordance with certain embodiments of the present invention.

Printing device 500 includes an ink source 502 that is configured to hold ink. Ink source 502 is coupled a pump 506 through a conduit 504 in a manner that allows the ink held in ink source 502 to be withdrawn via conduit 104. Conduit 508 is further coupled to an inlet of a liquid-gas separator 110 (or 110'). Liquide-gas separator 110 (or 110') is configured to at least substantially separate air that may be present in the urged flowing ink. The separated air exits liquid-gas separator 110 (or 110') through an air outlet 516, whereby the air is released into the atmosphere.

liquid-gas separator 110 (or 110') also includes a liquid outlet (not shown) that is coupled to conduit 512. The ink having been separated from the air continues to be urged by pump 506 through conduit 512 to a printhead 514. Printhead 514 is configured to selectively eject droplets of the ink onto a medium (not shown) as part of printing operation.

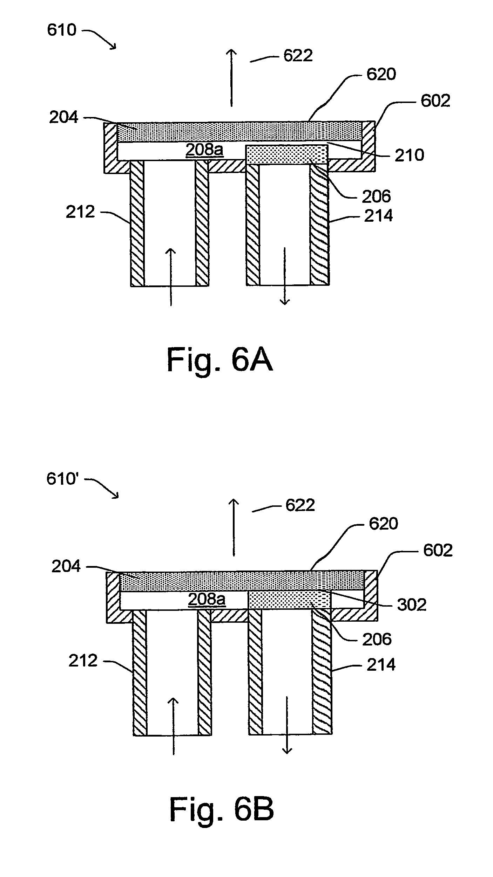

Figs. 6A-B are illustrative diagrams depicting cross-sectional views of two exemplary liquid-gas separators 610 and 610' liquid-gas separator 610 is similar to liquid-gas separator 110 and liquid-gas separator 610' is similar to liquid-gas separator 110'. In both of these examples, however, gas outlet 216 is inherently formed by housing 602 such that a back-side 620 of gas-permeable membrane 204 is directly exposed to a surrounding environment 622 which functionally serves as chamber 208b.

Although the above disclosure has been described in language specific to structural/functional features and/or methodological acts, it is to be understood that the appended claims are not limited to the specific features or acts descnbed. Rather, the specific features and acts are exemplary forms of implementing this disclosure.

## Claims

1. A liquid-gas separator (110', 610') comprising:
a gas-permeable membrane (204) establishing a boundary between a liquid-side chamber (208a) and a gas-side reg ion (620);
an inlet (212) configured to direct a flow of materials into said liquid-side chamber (208a), wherein said flow of materials includes at least one liquid and at least one gas;
a liquid outlet (214) having said liquid-permeable membrane cover (206), said liquid outlet (214) configured to direct said at least one liquid out of said liquid-side chamber (208a) through said liquid-permeable membrane cover (206) the liquid-gas separator being configured such that gas bubbles come into contact with the gas-permeable membrane (204) before or at about the same time that they would contact a liquid-permeable membrane cover (206); and wherein the gas-permeable membrane (204) is configured to provide a lower resistance for gas flow than the liquid-permeable membrane cover (206).
**characterized in that** a surface of said liquid-permeable membrane cover (206) contacts an op- H posing surface of said gas-permeable membrane (204) at a contact interface (302), and
**in that** the gas-permeable membrane is configured to flex or otherwise move in H response to liquid pressure within the liquid-side chamber (208a) thereby opening the contact interface (302) to allow liquid to flow from the liquid-side chamber (208a) through the liquid-permeable membrane (206) cover,

2. The liquid-gas separator (110', 610) as recited in Claim 1, wherein said gas-side region (620) is inside a gas-side chamber (208b) and further comprising:
a gas outlet (216) directing said at least one gas out of said gas-side chamber (208b) after passing through said gas-permeable membrane (204).

## Patentansprüche

1. Flüssigkeit-/Gasabscheider (110', 610'), umfassend:
eine gasdurchlässige Membran (204), die eine Grenze zwischen einer flüssigkeitsseitigen Kammer (208a) und einem gasseitigen Bereich (620) bildet;
einen Einlass (212), der eingerichtet ist, um einen Materialstrom in die flüssigkeitsseitige Kammer (208a) zu lenken, wobei der Materialstrom zumindest eine Flüssigkeit und zumindest ein Gas enthält;
einen Flüssigkeitsauslass (214) mit der flüssigkeitsdurchlässigen Membranabdeckung (206), wobei der Flüssigkeitsauslass (214) eingerichtet ist, um die zumindest eine Flüssigkeit aus der flüssigkeitsseitigen Kammer (208a) durch die flüssigkeitsdurchlässige Membranabdeckung (206) hinaus zu lenken, wobei der Flüssigkeits-/Gasabscheider derart eingerichtet ist, dass Gasblasen mit der gasdurchlässigen Membran (204) in Kontakt gelangen, bevor dem oder ungefähr zu dem gleichen Zeitpunkt, an dem sie eine flüssigkeitsdurchlässige Membranabdeckung (206) berühren würden; und
wobei die gasdurchlässige Membran (204) eingerichtet ist, um einen geringeren Widerstand für den Gasstrom als die flüssigkeitsdurchlässige Membranabdeckung (206) bereitzustellen;
**dadurch gekennzeichnet, dass**
eine Fläche der flüssigkeitsdurchlässigen Membranabdeckung (206) eine gegenüberliegende Fläche der gasdurchlässigen Membran (204) an einer Kontaktschnittstelle (302) berührt, und dadurch, dass
die gasdurchlässige Membran eingerichtet ist, um sich als Reaktion auf Flüssigkeitsdruck innerhalb der flüssigkeitsseitigen Kammer (208a) zu biegen oder anderweitig zu bewegen, wodurch die Kontaktschnittstelle (302) geöffnet wird, um zu ermöglichen, dass Flüssigkeit von der flüssigkeitsseitigen Kammer (208a) durch die flüssigkeitsdurchlässige Membranabdeckung (206) fließt.

2. Flüssigkeits-/Gasabscheider (110', 610) nach Anspruch 1, wobei sich der gasseitige Bereich (620) innerhalb einer gasseitigen Kammer (208b) befindet, und ferner umfassend:
einen Gasauslass (216), der das zumindest eine Gas aus der gasseitigen Kammer (208b) hinaus lenkt, nachdem es die gasdurchlässige Membran (204) passiert hat.

## Revendications

1. Séparateur liquide-gaz (110', 610') comprenant :
- une membrane perméable aux gaz (204) établissant une limite entre une chambre côté liquide (208a) et une région côté gaz (620) ;
- une entrée (212) configurée pour diriger un écoulement de matières dans ladite chambre côté liquide (208a), ledit écoulement de matières comprenant au moins un liquide et au moins un gaz ;
- une sortie de liquide (214) ayant un recouvrement formé d'une membrane perméable aux liquides (206), ladite sortie de liquide (214) étant configurée pour diriger ledit au moins un liquide hors de ladite chambre côté liquide (208a) à travers ledit recouvrement formé de la membrane perméable aux liquides (206), le séparateur gaz-liquide étant configuré de telle sorte que des bulles de gaz viennent en contact avec la membrane perméable aux gaz (204) avant le moment ou environ au même moment où elles entreraient en contact avec le recouvrement formé de la membrane perméable aux liquides (206) ; et
- la membrane perméable aux gaz (204) étant configurée pour fournir une résistance inférieure pour un écoulement de gaz au recouvrement formé de la membrane perméable aux liquides (206),
**caractérisé par le fait qu'**une surface dudit recouvrement formé de la membrane perméable aux liquides (206) vient en contact avec une surface opposée de ladite membrane perméable aux gaz (204) à une interface de contact (302), et **par le fait que** la membrane perméable aux gaz est configurée pour fléchir ou autrement se déplacer en réponse à une pression de liquide à l'intérieur de la chambre côté liquide (208a), permettant ainsi d'ouvrir l'interface de contact (302) pour permettre au liquide de s'écouler de la chambre côté liquide (208a) à travers le recouvrement formé de la membrane perméable aux liquides (206).

2. Séparateur liquide-gaz (110', 610) selon la revendication 1, dans lequel ladite région côté gaz (620) est à l'intérieur d'une chambre côté gaz (208b) et comprenant en outre:
une sortie de gaz (216) dirigeant ledit au moins un gaz hors de ladite chambre côté gaz (208b) après passage à travers ladite membrane perméable aux gaz (204).
